# EUROPEAN PATENT SPECIFICATION

(11) **EP 3 854 363 B1**
(45) Date of publication and mention of the grant of the patent: **30.11.2022**
(21) Application number: 19863724.1
(22) Date of filing: 11.07.2019
(51) Int. Cl.: A61F 13/511, A61F 13/513

(54) **ABSORBENT ARTICLE AND TOP SHEET**
ABSORBIERENDER ARTIKEL UND DECKLAGE
ARTICLE ABSORBANT ET COUCHE SUPÉRIEURE

(30) Priority: 18.09.2018 JP 2018173444
(43) Date of publication of application: 28.07.2021
(73) Proprietor: DAIO PAPER CORPORATION, Shikokuchuo-shi Ehime 799-0492 (JP)
(72) Inventor: SUYAMA, Junnosuke, Sakura-shi, Tochigi 329-1411 (JP)
(74) Representative: Epping - Hermann - Fischer
(86) International application number: PCT/JP2019/027571
(87) International publication number: WO 2020/059265

(56) References cited:
- EP-A1- 3 123 993
- JP-A- 2008 136 564
- JP-A- 2012 239 720
- JP-A- 2013 248 309
- JP-A- 2018 086 170

## Description

### TECHNICAL FIELD

The present invention relates to an absorbent article and a top sheet.

### BACKGROUND ART

As absorbent articles such as sanitary napkins, panty liners, and incontinence pads, an absorbent article that includes a liquid permeable top sheet, a liquid impermeable back sheet, and an absorbent body interposed between the liquid permeable top sheet and the liquid impermeable back sheet is known. A nonwoven fabric formed by accumulating fibers is often used as the top sheet. It is known that the top sheet is subjected to a shaping process by heat, pressure, and the like, in order to improve the diffusibility of body fluids and enhance texture.

For example, Patent Document 1 describes an absorbent article that includes a liquid permeable top sheet formed of two layers of a cushion layer and a skin contact layer. In the absorbent article described in Patent Document 1, heat-embossed portions are formed on the entire top sheet. The heat-embossed portions are arranged in a staggered pattern, and the long-side direction of the heat-embossed portions corresponds to the transverse direction of the absorbent article. In addition, the distance between two adjacent heat-embossed portions in each of the long-side direction and the short-side direction of the heat-embossed portions is twice or more the thickness of a member that is subjected to heat embossing.

### [RELATED-ART DOCUMENTS]

### [PATENT DOCUMENTS]

Japanese Patent No. 4953613 (Patent Document

Further document JP 2012 239720 A relates to absorbent articles such as sanitary napkins, disposable diapers, and incontinence pants.

Further document JP 2013 248309 A relates to absorbent articles such as sanitary napkins, panty liners, and light incontinence liners for absorbing menstrual blood, vaginal discharge, urine, and the like.

Further document JP 2018 086170 A relates to an absorbent article such as a sanitary napkin, a panty liner, an incontinence pad, and more specifically, side sheets are disposed along the longitudinal direction on both sides on the skin side, and embossing is provided on the side sheets.

### SUMMARY OF THE INVENTION

### PROBLEM TO BE SOLVED BY THE INVENTION

However, in the absorbent article described in Patent Document 1, long sides and short sides of the heat-embossed portions are arranged in parallel in respective directions, and thus, the heat-embossed portions tend to be located in close proximity. In particular, body fluids may be readily held in regions between heat-embossed portions that are arranged in the short-side direction of the heat-embossed portions, and may remain in the top sheet. Further, in the absorbent article described in Patent Document 1, the appearance of the heat-embossed portions is not taken into consideration, and the requirements of users who attach importance to the design are not sufficiently met.

In view of the above, it is an object of one aspect of the present invention to provide an absorbent article that prevents body fluids from remaining in a top sheet and has excellent design.

### MEANS TO SOLVE THE PROBLEM

According to one embodiment of the present invention, an absorbent article includes a top sheet, a liquid impermeable back sheet, and an absorbent body interposed between the top sheet and the back sheet. Welded portions are formed on at least a part of the top sheet. The welded portions constitute a plurality of units, and each of the units includes rod portions that are arranged radially in planar view. The distance between adjacent units is greater than the diameter of a smallest virtual circle inscribed in the rod portions included in each of the units. A short side of a first rod portion included in a first unit of the plurality of units and a short side of a first rod portion of a second unit of the plurality of units, the second unit being not the first unit, are in a first virtual straight line, and a short side of a second rod portion included in the first unit and a short side of a second rod portion are in a second virtual straight line, such that when a force causing the top sheet to twist is applied, a plurality of units can deform in the same direction along the first or second virtual straight line.

### EFFECTS OF THE INVENTION

According to an aspect of the present invention, an absorbent article that prevents body fluids from remaining in a top sheet and has excellent design can be provided.

### BRIEF DESCRIPTION OF THE DRAWINGS

FIG. 1 illustrates a partially cutaway plan view and a cross-sectional view of an absorbent article according to an embodiment of the present invention;
FIG. 2 is a diagram illustrating a pattern of welded portions formed on a top sheet according to an embodiment of the present invention;
FIG. 3 is a diagram illustrating variation examples of welded portions;
FIG. 4 is a diagram illustrating a variation example of a welded portion;
FIG. 5 is a diagram illustrating a variation example of a pattern of welded portions; and
FIG. 6 is an enlarged view of a part of the pattern of FIG. 5.

### MODE FOR CARRYING OUT THE INVENTION

In the following, embodiments of the present invention are described below with reference to the accompanying drawings. In the drawings, unless otherwise indicated, the same or corresponding elements may be denoted by the same reference numerals, and a duplicate description thereof may be omitted. In order to facilitate understanding of the present invention, the depiction of certain elements in the figures may be enlarged or reduced.

According to an embodiment of the present invention, an absorbent article that includes a top sheet having a predetermined configuration is provided. Examples of the absorbent article include a sanitary napkin, a panty liner, an incontinence pad, and a disposable diaper. Further, according to an embodiment of the present invention, a top sheet is provided.

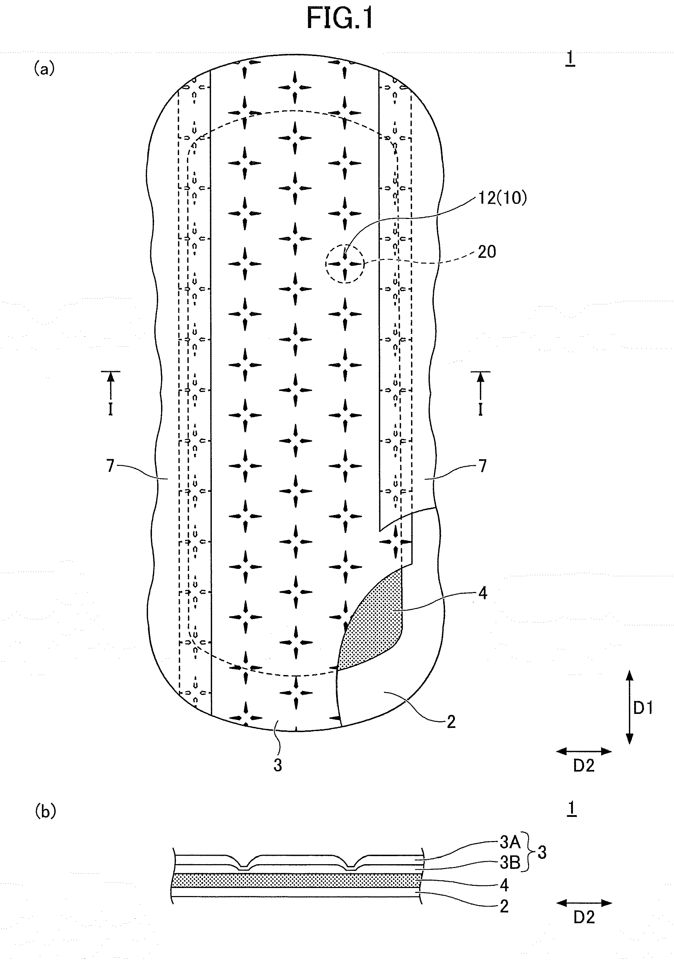

FIG. 1 (a) is a partially cutaway plan view of an absorbent article 1 according to an embodiment. FIG. 1 (b) is a cross-sectional view of the absorbent article 1 taken through I-I of FIG. 1 (a) (side portions of the absorbent article 1 are not depicted).

As illustrated in FIG. 1 (a) and (b), the absorbent article 1 is a planar article that includes a liquid impermeable back sheet 2, a liquid permeable top sheet 3, and an absorbent body 4 disposed between the back sheet 2 and top sheet 3. When the absorbent article 1 is worn, the top sheet 3 contacts the skin (that is, the top sheet 3 is on the skin side or the top surface side), and the back sheet 2 is attached to underwear (that is, the back sheet 2 is on the underwear side or the back surface side).

As illustrated in FIG. 1 (a), the absorbent article 1 has an elongated shape as a whole. That is, the absorbent article 1 has a predetermined length in a first direction (longitudinal direction) D1 and a predetermined width in a second direction (width direction) D2 that is perpendicular to the first direction D1. The width of the absorbent article 1 may be approximately uniform, or may vary in the first direction (longitudinal direction) D1. When the absorbent article 1 is worn, the absorbent article 1 is attached to a crotch portion of the underwear such that the longitudinal direction D1 of the absorbent article 1 is in the front-back direction of the body. In this manner, the absorbent article 1 can be brought into contact with the body.

The absorbent body 4 has a size and a shape such that the absorbent body 4 does not protrude from the back sheet 2 in planar view. At both end portions in the longitudinal direction of the absorbent body 4, outer end portions of the back sheet 2 and the top sheet 3 are bonded to each other with an adhesive such as a hot-melt adhesive or with an adhesive means such as a heat seal or an ultrasonic seal. Further, side nonwoven fabrics 7, 7 are provided at side portions of the top sheet 3 along the longitudinal direction D1. The side nonwoven fabrics 7, 7 and the back sheet 2 are bonded to each other with an adhesive means as described above, at side portions (both end portions in the width direction) of the absorbent body 4. A slip preventive portion 9 for securing the absorbent article 1 to the underwear may be provided on the back surface of the back sheet 2.

The back sheet 2 is a sheet that prevents body fluids (such as menstrual blood, vaginal discharge, and urine), having permeated the absorbent body 4 and reached the back surface of the back sheet 2, from being transferred to the underwear. The back sheet 2 may use a sheet material such as an olefin resin sheet formed of polyethylene or polypropylene and having at least a water shielding property. Alternatively, the back sheet 2 may use a polyethylene-sheet-laminated nonwoven fabric or a nonwoven fabric layered sheet having a waterproof film interposed therebetween such that impermeability is substantially ensured. Further, a material having moisture permeability is desirably used to prevent stuffiness. As such a water shielding and permeable sheet material, a microporous sheet is preferably used. The microporous sheet is obtained by forming a sheet by melting and kneading inorganic filler with an olefin resin such as polyethylene and polypropylene, and subsequently stretching the sheet in one axial direction or two axial directions.

The top sheet 3 is a liquid permeable sheet that causes body fluids to quickly pass through and to be transferred to the absorbent body 4 layered on the back surface of the top sheet 3. As the top sheet 3, a nonwoven fabric composed of synthetic or natural fibers is preferably used. Alternatively, a woven fabric laminated with a porous plastic sheet may be used.

Examples of fibers constituting the top sheet 3 include synthetic fibers such as olefin such as polyethylene or polypropylene, polyester such as polyethylene terephthalate or polybutylene terephthalate, polyamide such as nylon 6 or nylon 66; regenerated fibers such as rayon or cuprammonium rayon; mixed fibers thereof; and natural fibers such as cotton. Further, composite fibers such as core-in-sheath fibers having a high-melting-point fiber as a core and a low-melting-point fiber as a sheath, side-by-side fibers, or split fibers may be used. These fibers can be used alone or in combination with two or more kinds.

Further, examples of a method for processing a nonwoven fabric include a spunlace method, a spunbond method, a thermal bond method, a melt blown method, and a needle punch method. Among the above methods, the spunlace method is preferred in terms of flexibility, the spunbond method is preferred because a nonwoven fabric with high drape properties can be manufactured, and the thermal bond method is preferred because a soft nonwoven fabric with high bulkiness can be manufactured.

Further, the top sheet 3 may be subjected to treatment for providing hydrophilicity or hydrophobicity. The treatment may be performed by mixing a hydrophilic agent or a hydrophobic agent with a fiber material beforehand, or applying a hydrophilic agent or a hydrophobic agent to the surface of a nonwoven fabric beforehand.

The top sheet 3 may have a multi-layer structure in which a first top sheet (top sheet) 3A and a second top sheet (second sheet) 3B are laminated (FIG. 1 (b)). The first top sheet and the second top sheet may have the same configuration, or may have different configurations. The first top sheet 3A preferably functions to diffuse body fluids in the thickness direction of the absorbent article 1, that is, the first top sheet 3A preferably functions to diffuse body fluids into the absorbent body 4. The second top sheet 3B preferably functions to diffuse body fluids in the plane direction of the absorbent article 1. Further, if the top sheet 3 is subjected to a shaping process such as a welding process, the first top sheet 3A and the second top sheet 3B are preferably formed of the same material or include the same material. In this manner, fibers included in the first top sheet 3A and the second top sheet 3B can be readily bonded to each other, thus enabling the shaping process even under relatively weak conditions.

The above-described materials may be used as the material of the first top sheet 3A and the material of second top sheet 3B. Preferably, hydrophilic nonwoven fabrics formed by the thermal bond method or the spunbond method are used as the first top sheet 3A and the second top sheet 3B. More preferably, nonwoven fabrics formed by the thermal bond method are used because the nonwoven fabrics formed by the thermal bond method include fine, bulky fibers and have enhanced texture. If the first top sheet (top sheet) 3A and the second top sheet (second sheet) 3B are formed of different materials, the first top sheet 3A is preferably formed of a thermally bonded nonwoven fabric, and the second top sheet 3B is preferably formed of a spunbonded nonwoven fabric. Accordingly, the top sheet 3 is configured such that the fiber density of the first top sheet 3A is lower than the fiber density of the second top sheet 3B. With this configuration, body fluids can readily flow from the first top sheet 3A into the second top sheet 3B by capillary action, thus allowing body fluids discharged onto the first top sheet 3A to be rapidly directed into the absorbent body 4. In addition, the user can recognize that body fluids are securely absorbed because body fluids are not readily seen from the surface.

Note that the top sheet 3 may include three or more layers. In this case, the three or more layers may have the same configuration, or may have different configurations. Further, the above-described materials may be used as the materials of the layers.

The material of the absorbent body 4 is not particularly limited, and preferably includes cotton-like pulp and a water-absorptive polymer. Examples of the water-absorptive polymer include a superabsorbent polymer (SAP), a superabsorbent polymer fiber (SAF), and a combination thereof. Examples of the pulp include cellulose fibers such as dissolving pulp and chemical pulp made from wood, and synthetic cellulose fibers such as rayon and acetate. Materials of the chemical pulp include softwood pulp and hardwood pulp. Because of a long fiber length, the softwood pulp is preferably used.

The absorbent body 4 is preferably manufactured by a fiber lamination method or an air laid method. In order to maintain the shape of the absorbent body 4, the absorbent body 4 may be surrounded by a colored or an uncolored (white) encapsulating sheet made of a crepe paper sheet or a nonwoven fabric.

As the side nonwoven fabrics 7, water-repellent nonwoven fabrics or hydrophilic non-woven fabrics may be used in order to prevent the permeation of body fluids or to enhance texture. The side nonwoven fabrics 7 may be composed of natural fibers, synthetic fibers, or regenerated fibers. For water repellency, the side nonwoven fabrics 7 may be coated with a water-repellent agent such as a silicon-based or a paraffin-based water-repellent agent.

According to an embodiment of the present invention, the top sheet 3 is subjected to a shaping process in which three-dimensional recesses and projections are formed. Subjecting the top sheet 3 to the shaping process improves the function of directing body fluids, enhances texture by reducing the area of the top sheet 3 that contacts the skin, and also increases the stiffness of the top sheet 3. In addition, recessed portions (shaped portions) formed by the shaping process can represent predetermined shapes that can be visually recognized, thus improving the design and appearance of the top sheet 3.

The shaping process may be a process in which the thickness of a sheet is locally varied by heating and/or applying pressure to the sheet or by using an agent to locally denature the material of the sheet. The shaping process is preferably a process (welding process) in which a plurality of welded portions are formed spaced apart from each other. The welded portions are portions where fibers included in the sheet are at least partially welded (fused) and the bulk density is increased. The thicknesses of the welded portions are smaller than the thicknesses of portions surrounding the welded portions. That is, the welded portions are recessed portions or dented portions. The welded portions may be formed by what is known as heat embossing that uses a roller or the like to apply heat and pressure.

If the top sheet 3 includes a single layer, fibers included in the single layer are welded, thereby forming welded portions. If the top sheet 3 includes two or more laminated layers, fibers between the laminated layers are welded, or fibers included in the same layer and fibers between the laminated layers are welded, thereby forming welded portions.

According to an embodiment of the present invention, welded portions can be formed over at least a part of the top sheet 3. That is, a plurality of welded portions, spaced apart from each other, may be formed over a part of the surface of the top sheet 3, or may be formed over the entire surface of the top sheet 3 as illustrated in FIG. 1 (a). In order to form welded portions over a part of the top sheet 3, the welded portions may be formed in regions where the absorbent body 4 is layered and/or regions that are not covered by the side nonwoven fabrics 7. Further, welded portions are preferably formed in a region corresponding to a body fluid discharge region and in the vicinity of the body fluid discharge region.

The ratio of the area (hereinafter may be referred to as the "area ratio") of the welded portions of the above-described part of the top sheet 3 to the area of the top sheet 3, which is taken as 100%, may be set to 1% to 50%. By setting the area ratio of the welded portions to 1% or more, an effect of directing body fluids can be improved. In addition, by setting the area ratio of the welded portions to 50% or less, an excessive increase in the stiffness of the top sheet 3 and in the stiffness of the absorbent article 1 can be prevented, and also body fluids can be prevented from being excessively held in the top sheet 3. As a result, hindering the diffusion of body fluids can be prevented.

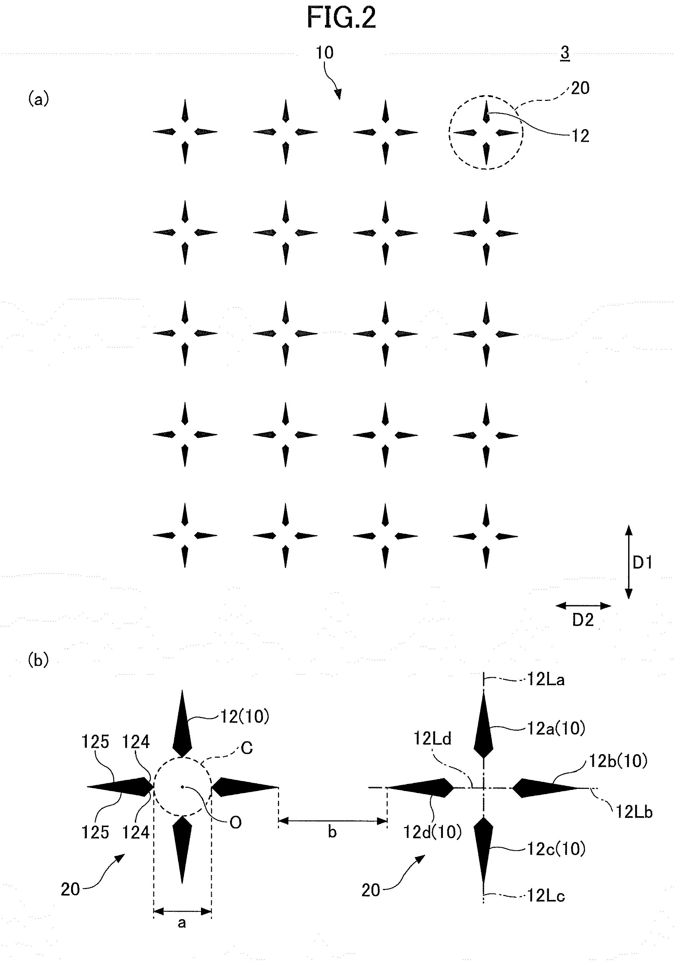

In the following, welded portions formed on the top sheet 3 will be described in more detail. FIG. 2 (a) depicts an enlarged plan view of a part of the top sheet 3 according to an embodiment of the present invention. A pattern of welded portions 10 of FIG. 2 (a) is different from that of FIG. 1. Further, FIG. 2 (b) depicts a further enlarged view of welded portions. In FIG. 2, the welded portions 10 are indicated in black.

As illustrated in FIG. 2 (a) and (b), the welded portions 10 constitute a plurality of units 20. Each of the units 20 includes portions (rod portions 12) that have rod shapes in planar view and are arranged radially in planar view. As used herein, the "rod shape" means a two-dimensional shape of a rod projected in a direction perpendicular to the long-side direction of the rod, that is, an elongated shape in which the length is greater than the width. The width of the shape (shape formed by the outline) of each of the rod portions 12 may be uniform, but is not required to be uniform. In addition, one end or both ends in the long-side direction of each of the rod portions 12 may be rounded or pointed. Further, the rod portions 12 are not required to have a linear shape, and may have a bent shape or a curved shape.

As described above, because the welded portions each include the rod portions 12, body fluids can be readily directed along the long sides of the rod portions 12 to regions where no rod portions 12 are formed. Accordingly, body fluids can be readily directed in a desired direction on the top sheet 3 and/or in the top sheet 3 in accordance with the arrangement of the plurality of rod portions 12.

The shape of each of the rod portions 12 in planar view may be an angular shape, or may be a rounded shape such as an elongated elliptical shape. As long as each of the rod portions 12 can be perceived as having a rod shape, the rod portions 12 may be each formed in a recessed shape. Specifically, each of the rod portions 12 may have a polygonal shape such as a triangle shape or a quadrilateral shape. Examples of the quadrilateral shape include a rectangular shape, a parallelogram shape (including a diamond shape), and a trapezoidal shape. If each of the rod portions 12 has a polygonal shape, one vertex of each of the polygonal-shaped rod portions 12 is disposed facing the center of a corresponding unit 20. In this manner, body fluids discharged within the unit 20 can be rapidly directed to the outside of the unit 20 along the two sides of the vertex of each of the rod portions 12.

In the examples illustrated in FIG. 2 (a) and (b), the rod portions 12 have kite shapes. That is, each of the rod portions 12 is a quadrilateral in which the length of each side is equal to the length of one of two adjacent sides, or is a quadrilateral formed by two isosceles triangles. When the rod portions 12 have kite shapes, body fluids can be readily directed along the longer sides of the rod portions 12. Therefore, by arranging the rod portions 12 in accordance with a desired direction in which to direct body fluids, the direction of body fluids can be readily controlled throughout the top sheet 3.

In the example illustrated in FIG. 2 (b), a kite-shaped rod portion 12 is formed such that short sides 124, 124 face the center of a unit 20, and the vertex of the two short sides 124, 124 face the center of the unit 20. That is, the length (in the long-side direction) extending from a portion where the kite-shaped rod portion 12 has the maximum width to the inner end portion of the kite-shaped rod portion 12 is less than the length extending from the portion where the kite-shaped rod portion 12 has the maximum width to the outer end portion of the kite-shaped rod portion 12. Accordingly, body fluids discharged in the vicinity of the center of the unit 20 can be rapidly directed along the two short sides 124, 124 of the kite-shaped rod portion 12, and can be further directed along two long sides 125, 125 from the center of the unit 20 to the outside of the unit 20.

The aspect ratio of the rod portion 12 is preferably approximately 2 to 10, and is more preferably approximately 2.5 to 6. As used herein, the "aspect ratio" is the value of the ratio of the length of the rod portion 12 to the width of the rod portion 12. That is, the "aspect ratio" is the value of the ratio of the length in the long-side direction of the rod portion 12 to the length in the short-side direction perpendicular to the long-side direction of the rod portion 12. If the rod portion 12 has a shape that varies in width, the aspect ratio may be the value of the ratio of the maximum length to the maximum width of the rod portion 12. Further, if the rod portion 12 is curved, the aspect ratio may be the value of the ratio of the distance between both ends of the rod portion 12 to the maximum width of the rod portion 12.

The aspect ratio of 2 or more can facilitate directing bodily fluids along the long edge portions or the long sides of the rod portion 12. The aspect ratio of 10 or less can prevent the flow of body fluids in the short-side direction of the rod portion 12 from being hindered.

The length (in the long-side direction) of the rod portion 12 may be 1.0 mm to 10 mm. The maximum width of the rod portion 12 may be 0.5 mm to 2.0 mm.

As illustrated in FIG. 2 (a) and (b), a plurality of rod portions 12 included in a corresponding unit 20 are arranged radially. That is, the rod portions 12 included in the unit 20 are arranged such that the long sides of the rod portions 12 extend outward from the center of the unit 20 in respective directions. For example, one end of each of the rod portions 12 is disposed facing the center of the unit 20. That is, when a centerline is assumed to be drawn through the center of each of the rod portions 12 (a centerline along the long-side direction of each of the rod portions 12, or a line that halves the width of each of the rod portions 12), the rod portions 12 are preferably arranged such that the centerlines of the rod portions 12 meet at the center of the unit 20. In this case, the centerlines are preferably arranged at equal angular intervals.

If one unit 20 includes three or more rod portions 12, the three or more rod portions 12 included in the one unit can be arranged such that the centerlines of the three or more rod portions 12 intersect at one point. For example, as indicated by a unit 20 on the right side of FIG. 2 (b), centerlines 12La, 12Lb, 12Lc, and 12Ld of rod portions 12a, 12b, 12c, and 12d extend at equal angular intervals and pass through the center point of the unit 20. In the example illustrated in FIG. 2 (b), centerlines adjacent to each other intersect at an angle of approximately 90 degrees. The centerlines 12La and 12Lc of the opposite rod portions 12a and 12c are in a straight line, and the centerlines 12Lb and 12Ld of the opposite rod portions 12b and 12d are in a straight line.

With the above-described radial arrangement, excessive proximity of a plurality of rod portions 12 in a unit 20 can be prevented. For example, arranging two or more rod portions 12 in parallel or arranging three or more rod portions 12 in parallel can be prevented. In this manner, a plurality of rod portions 12 can be prevented from being arranged too close to each other.

Further, as indicated by a unit 20 on the left side of FIG. 2 (b), a plurality of rod portions 12 included in a unit 20 are arranged such that a virtual circle C inscribed in the rod portions 12 can be drawn. Therefore, the rod portions 12 can maintain a predetermined distance from each other, and can be prevented from being arranged too close to each other. Because the rod portions 12 are not arranged too close to each other, an excessively large amount of body fluids is not held in the top sheet 3. As a result, body fluids can be prevented from remaining in the top sheet 3 (that is, the diffusion of body fluids in welded portions and in the vicinities of the welded portions can be prevented from being hindered).

Further, a plurality of units 20 are formed on the top sheet 3, and each of the units 20 includes a plurality of rod portions 12. In the example of FIG. 2 (a), the plurality of units 20 are arranged in a grid pattern. The absorbent article 1 is formed such that the vertical and horizontal directions of the grid pattern correspond to the longitudinal direction D1 and the width direction D2 of the absorbent article 1 illustrated in FIG. 2 (a). However, the relationship between the longitudinal direction D1 and the width direction D2 of the absorbent article 1 versus the pattern of the plurality of units 20 of the top sheet 3 is not limited to the example illustrated in FIG. 2 (a).

As illustrated in FIG. 2 (a), rod portions 12 included in one unit 20 are not shared with rod portions 12 included in another adjacent unit 20. In other words, one unit includes a plurality of rod portions 12, and another unit includes a plurality of rod portions 12 other than the rod portions 12 included in the one unit.

A plural number of rod portions 12 may be included in one unit 20. Preferably, two to five rod portions 12 may be included in one unit 20. By setting the number of rod portions 12 included in a unit to two or more, the unit can represent a highly designed shape, and also body fluids can be readily directed along the rod portions 12. Further, by setting the number of rod portions 12 included in a unit to five or less, the rod portions 12 can be prevented from being arranged too close to each other, thus preventing an excessive increase in stiffness of the unit 20 and in the vicinity thereof.

Further, as illustrated in FIG. 2 (b), the distance b between units 20 is greater than the diameter a of the smallest virtual circle C inscribed in rod portions 12 included in each of the units 20. As used herein, the "distance b between units 20" refers to the minimum distance between a rod portion 12 included in one unit 20 and a rod portion 12 included in another adjacent unit 20.

If three rod portions 12 are included in one unit, one virtual circle can be inscribed in the rod portions 12. Thus, the one virtual circle is set to be the smallest virtual circle C. Conversely, if four or more rod portions 12 are included in one unit, a plurality of virtual circles may be inscribed in the rod portions 12. Thus, the smallest virtual circle of the plurality of virtual circles is set to be the smallest virtual circle C. As in the examples illustrated in FIG. 2 (a) and (b), even when four or more rod portions 12 are included in one unit, one virtual circle can be inscribed in the rod portions 12 if the center O of the virtual circle is located at an equal distance from all the rod portions 12. Thus, the one virtual circle is set to be the smallest virtual circle C.

The above-described relationship between the distance between units 20 and the size of a virtual circle inscribed in rod portions 12 allows the units 20 to be sufficiently spaced apart from each other. Therefore, a plurality of rod portions 12 included in one unit 20 can be integrally recognized by the user. In addition, because each unit 20 can represent a predetermined shape, a variety of designs may be possible.

The diameter a of the smallest virtual circle C may be 0.5 mm to 5 mm. By setting the diameter a to 0.5 mm or more, a more reliable pathway of body fluids can be formed at the center of a unit 20. In addition, a plurality of rod portions 12 are prevented from being arranged too close to each other, thus preventing an excessive increase in stiffness. By setting the diameter a to 5 mm or less, a plurality of rod portions 12 included in one unit 20 can be formed integrally, and the shape formed by the one unit 20 can be readily recognized.

More specifically, the distance b between two adjacent units 20 may be 0.6 mm to 15 mm, and may preferably be 3 mm to 12 mm. Further, the value of the ratio (b/a) of the distance b to the diameter a, which exceeds 1, is preferably 1.1 to 10, and is more preferably 2 to 7. By setting the above value of the ratio to 1.1 or more, a plurality of rod portions 12 included in a unit 20 can be formed integrally, and the shape formed by the unit 20 can be readily recognized. By setting the above value of the ratio to 10 or less, body fluids can be directed along a plurality of units 20, that is, body fluids can be directed from rod portions 12 included in one unit 20 to rod portions 12 included in another unit 20.

From the viewpoint of design, the predetermined shape represented by a unit 20 is not limited to a geometric shape, and may be any shape that reminds people of a plant, an animal, a daily necessity, or an ornament. Examples of the predetermined shape include a flower, a leaf, a star, a snow crystal, and a ribbon (including a tied-ribbon shape). In the examples illustrated in FIG. 1 and FIG. 2, each unit 20 is formed in a shape that reminds people of a star or flashing light beams.

As described above, in the present embodiment, the predetermined shape can be represented by each unit 20 (welded portion 10) that includes rod portions 12 disposed spaced apart from each other. Accordingly, the total area of welded portions can be reduced, as compared to when a predetermined shape is represented by each continuous welded portion.

In order to improve the visibility of welded portions by placing priority on the design of the welded portions, the welding process (shaping process) may be performed under strengthened conditions such as high temperature and high pressure conditions. Further, such processing conditions may also be strengthened by the material of the top sheet 3 or by a combination of the material of the first top sheet 3A and the material of the second top sheet 3B. In such a case, fibers may be excessively welded, and as a result, a part of or the entirety of the welded portions may become non-porous. If the welded portions become non-porous, body fluids would not readily pass through the top sheet 3, and would remain in the welded portions and in the vicinities of the welded portions of the top sheet 3.

Conversely, in the present embodiment, the total area of welded portions can be reduced as compared to when a predetermined shape is represented by each continuous welded portion. Therefore, even when the welded portions are formed under strengthened processing conditions, the top sheet and the absorbent article can ensure liquid permeability while having an enhanced design represented by the shapes of the welded portions.

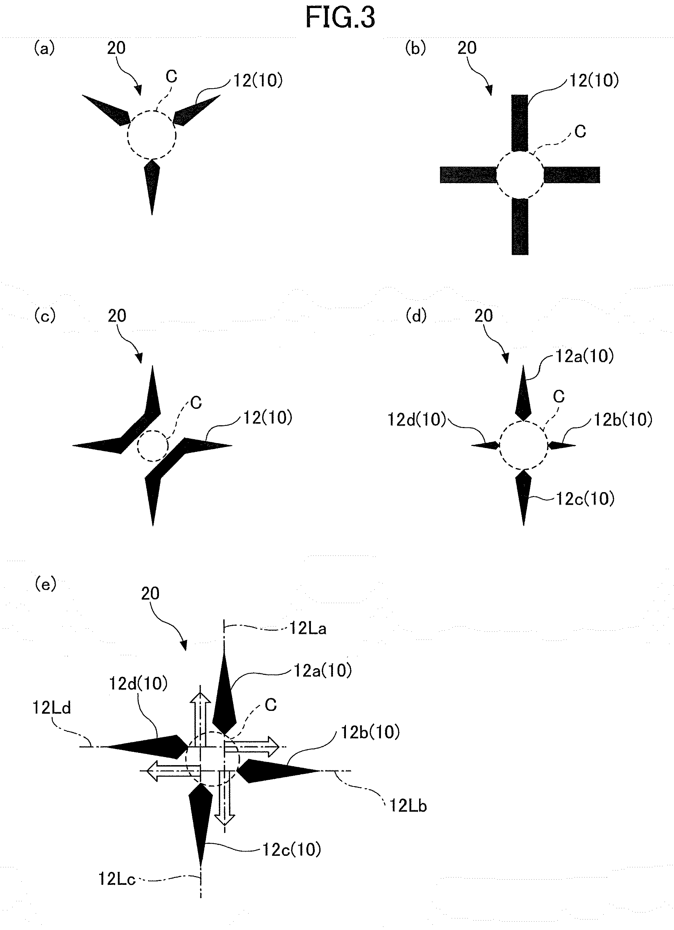

FIG. 3 (a) through (e) depict variation examples of rod portions 12 included in one unit. In FIG. 3 (a) through (e), one unit 20 includes a plurality of rod portions 12 that are radially arranged. In addition, the smallest virtual circle C inscribed in rod portions 12 is depicted in each of the variation examples.

A unit 20 of FIG. 3 (a) differs from FIG. 2 in that the number of rod portions 12 is three. In this example, the total welded region in one unit 20 can be reduced. Thus, the example in FIG. 3 (a) is preferable in terms of ensuring the flexibility of the top sheet 3.

The shapes of rod portions 12 included in a unit 20 of FIG. 3 (b) differs from those of FIG. 2. In this example, each of the rod portions 12 has a rectangular shape with an approximately uniform width. When each of the rod portions 12 has a rectangular shape with an approximately uniform width, the outlines of the rod portions 12 can be more clearly recognized. Accordingly, the visibility of the unit 20 can be improved, thus resulting in enhanced design.

In the example illustrated in FIG. 3 (c), the number of rod portions 12 included in a unit 20 is two. In addition, the rod portions 12 are not straight and are bent. When the rod portions 12 are bent as illustrated in FIG. 3 (c), or the rod portions 12 are curved, an angle formed by centerlines from both ends of each of the rod portions 12 to the center is preferably 80° or more.

In the examples illustrated in FIG. 3 (a) through FIG. 3 (c), a plurality of rod portions 12 included in a unit 20 have the same shape and size. However, as illustrated in FIG. 3 (d), a plurality of rod portions 12 included in a unit 20 may have different sizes. In the example illustrated in FIG. 3 (d), a total of four rod portions 12 including one pair of opposing rod portions 12a and 12c and another pair of opposing rod portions 12b and 12d are formed. Each of the pairs of opposing rod portions 12 has the same shape and size. However, the size of the one pair of opposing rod portions 12a and 12c differs from the size of the other pair of opposing rod portions 12b and 12d. Accordingly, body fluids can be readily directed in a particular direction. For example, if this variation example is applied to the top sheet 3 of the absorbent article 1, and the rod portions 12a and 12c are arranged in the longitudinal direction D1, body fluids can be readily directed in the longitudinal direction D1, and the leakage of body fluids in the width direction D2 can be minimized.

In the example illustrated in FIG. 3 (e), rod portions 12a, 12b, 12c, and 12d have kite shapes, similar to those illustrated in FIG. 2. However, centerlines 12La, 12Lb, 12Lc, and 12Ld of the rod portions do not intersect at one point. The centerlines 12La and 12Lc of the opposing rod portions 12a and 12c are in parallel. Similarly, the centerlines 12Lb and the 12Ld of the opposing rod portions 12b and 12d are in parallel.

In the example of FIG. 3 (e), body fluids can readily flow to the outside of a unit 20 as indicated by arrows. Accordingly, body fluids tend not to remain in the unit 20, thereby reducing body fluids remaining in the top sheet 3.

Note that any welded portions other than units 20 including rod portions 12 are not formed in the illustrated examples, but any other welded portions may be formed. For example, dot-shaped welded portions or welded portions having any other shape may be formed. However, no welded portions are preferably formed in virtual circles C in order to reduce the total area of welded portions.

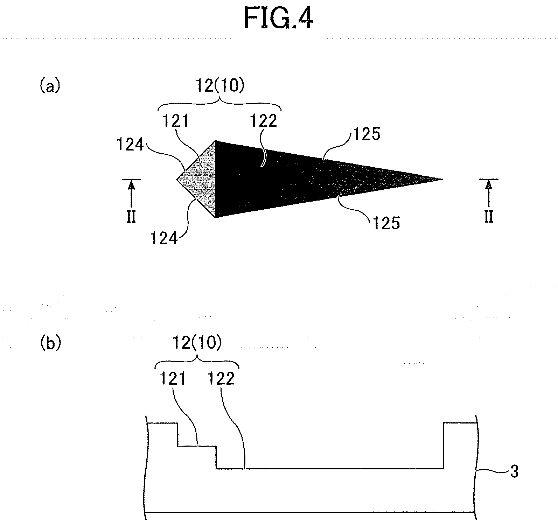

Further, FIG. 4 depicts a variation example of a rod portion 12. FIG. 4 (a) is an enlarged view of one kite-shaped rod portion 12 illustrated in FIG. 2 (a) and FIG. 2 (b). FIG. 4 (b) is a cross-sectional view of the rod portion 12 taken through II-II of FIG. 4 (a). As illustrated in FIG. 4 (a) and FIG. 4 (b), the rod portion 12 includes a shallow welded portion 121 having a relatively large thickness, which is namely a shallow recess, and a deep welded portion 122 having a relatively small thickness, which is namely a deep recess. The welded portion 12 with different thicknesses (or depths) can be formed by partially changing the conditions for welding the rod portion 12.

As illustrated in FIG. 4 (a) and (b), the kite-shaped rod portion 12 is formed such that the depth on the short sides 124, 124 is smaller than that on the long sides 125, 125. Accordingly, body fluids can be collected in the vicinity of the center of a unit 20, and readily directed to the outside of the unit 20.

As described above, the thickness of one rod portion 12 may be uniform, but is not necessarily uniform. Further, a plurality of rod portions 12 in one unit 20 may have the same thickness or may have different thicknesses.

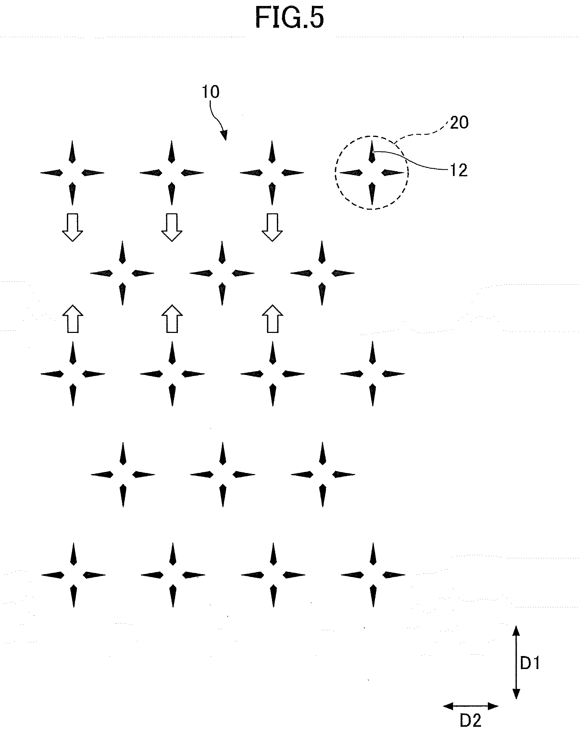

FIG. 5 depicts a variation example of an arrangement pattern of units 20. The pattern illustrated in FIG. 5 is similar to the pattern of the units 20 of the top sheet 3 used in the absorbent article 1 of FIG. 1 (a). In FIG. 2 (a), the plurality of units 20 are arranged in a grid pattern. In FIG. 5, the plurality of units 20 are arranged in a staggered pattern. The absorbent article 1 is formed such that the vertical and horizontal directions of the staggered pattern correspond to the longitudinal direction D1 and the width direction D2 of the absorbent article 1. However, the relationship between the longitudinal direction D1 and the width direction D2 of the absorbent article 1 versus the pattern of the plurality of units 20 is not limited to that illustrated in FIG. 5.

The above-described arrangement of the staggered pattern allows body fluids, directed along rod portions 12 to the outside of the units 20, to be further directed to regions where no welded portions are formed. Accordingly, body fluids can be readily diffused on and/or in the entire top sheet 3, as indicated by arrows in FIG. 5.

The grid pattern illustrated in FIG. 2 (a) and the staggered pattern illustrated in FIG. 5 are regular patterns. However, units 20 may be arranged in an irregular pattern and the distance b between adjacent units 20 may vary as long as the distance b is greater than the diameter a of the smallest virtual circle inscribed in rod portions 12 included in each unit 20.

Further, in the examples illustrated in FIG. 2 (a) and FIG. 5, the units 20 have the same configuration. However, the units 20 may have different configurations as long as the units 20 each include a plurality of rod portions 12 that are arranged radially, and the distance b between adjacent units 20 is greater than the diameter a of the smallest virtual circle inscribed in the rod portions 12 included in each of the units 20. For example, the rod portions 12 may have different lengths with respect to differing units 20. In addition, the diameter a of the smallest virtual circle C inscribed in the rod portions 12 may differ with respect to differing units 20.

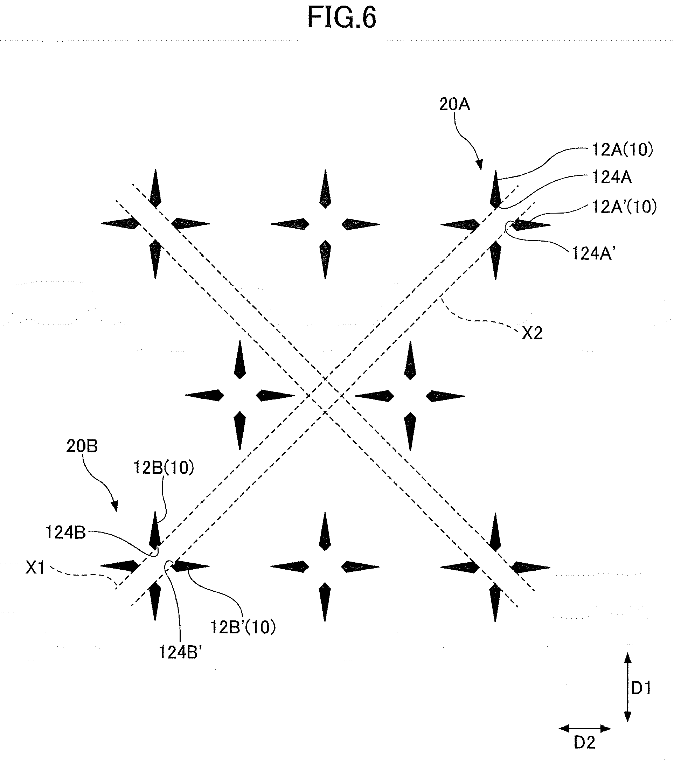

Further, in the pattern illustrated in FIG. 5, one short side of a kite-shaped rod portion 12 included in one unit 20 and one short side of a kite-shaped rod portion 12 included in another unit 20 are arranged in a straight line. FIG. 6 is an enlarged view of a part of the staggered pattern illustrated in FIG. 5.

As illustrated in FIG. 6, one short side 124A of a rod portion 12A included in a unit 20A and one short side 124B of a rod portion 12B of another unit 20B, which is not the unit 20A, are in a virtual straight line X1. Further, one short side 124A' of a rod portion 12A' included in the unit 20A and one short side 124B' of a rod portion 12B' are in a virtual straight line X2. Accordingly, when a force causing the top sheet 3 to twist is applied, a plurality of units can deform in the same direction (along a virtual straight line). Therefore, the top sheet 3 can deform following the movement of the wearer's body, thereby reducing discomfort when the absorbent article is worn.

As described above, the relationship between the longitudinal direction D1 and the width direction D2 of the absorbent article versus the pattern of welded portions is not particularly limited. In the arrangement illustrated in FIG. 6, the virtual straight lines X1 and X2 are at angles with respect to the longitudinal direction D1 and the width direction D2. Accordingly, even if the absorbent article twists about centerlines extending in the longitudinal direction when the absorbent article is worn, the absorbent article can readily deform following the twisting.

Further, if welded portions are formed by a compression drum, projections corresponding to the welded portions can be formed by linearly cutting the surface of the drum, that is, by cutting the surface of the drum along the virtual straight lines X1 and X2. Therefore, the manufacturing time and cost can be reduced. In this case, no welded portion is preferably formed in a region between the virtual straight line X1 and the virtual straight line X2, as illustrated in FIG. 6.

### DESCRIPTION OF THE REFERENCE NUMERALS

1 absorbent article
2 back sheet
3 top sheet
3A first top sheet (top sheet)
3B second top sheet (second sheet)
4 absorbent body
7 side nonwoven fabric
10 welded portion
12, 12a, 12b, 12c, 12d, 12A, 12A', 12B, 12B' rod portion
12La, 12Lb, 12Lc, 12Ld centerline
20 unit
121 shallow welded portion
122 deep welded portion
124, 124A, 124A', 124B, 124B' short side of kite-shaped rod portion
125 long side of kite-shaped rod portion
C smallest virtual circle
X1, X2 virtual straight line
D1 first direction (longitudinal direction of absorbent article 1)
D2 second direction (width direction of absorbent article 1)

## Claims

1. An absorbent article (1) comprising:
a top sheet (3); a liquid impermeable back sheet (2); and an absorbent body (4) interposed between the top sheet (3) and the back sheet (2),
wherein welded portions (10) are formed on at least a part of the top sheet (3),
the welded portions (10) constitute a plurality of units (20), each of the units (20) including rod portions (12) that are arranged radially in planar view,
a distance between adjacent units (20) is greater than a diameter of a smallest virtual circle (C) inscribed in the rod portions (12) included in each of the units (20),
**characterized in that**
a short side (124A) of a first rod portion (12A) included in a first unit (20A) of the plurality of units (20) and a short side (124B) of a first rod portion (12B) of a second unit (20B) of the plurality of units (20), the second unit (20B) being not the first unit (20A), are in a first virtual straight line (X1), and
a short side (124A') of a second rod portion (12A') included in the first unit (20A) and a short side (124B') of a second rod portion (12B') are in a second virtual straight line (X2), such that
when a force causing the top sheet (3) to twist is applied, a plurality of units (20) can deform in the same direction along the first or second virtual straight line.

2. The absorbent article (1) according to claim 1, wherein the top sheet (3) is formed by laminating a first top sheet (3A) and a second top sheet (3B), the first top sheet (3A) being on a skin side and the second top sheet (3B) being on an underwear side when the absorbent article (1) is worn.

3. The absorbent article (1) according to claim 1, wherein the diameter of the virtual circle is 0.5 mm to 5.0 mm.

4. The absorbent article (1) according to claim 1, wherein the number of the rod portions (12) included in each of the units (20) is 2 to 5.

5. The absorbent article (1) according to claim 1, wherein each of the rod portions (12) has an aspect ratio of 2 to 10.

6. The absorbent article (1) according to claim 1, wherein the units (20) are arranged in a staggered pattern.

7. The absorbent article (1) according to claim 1, wherein each of the rod portions (12) has a kite shape whose short sides (124) face a center of a corresponding unit (20).

8. The absorbent article (1) according to claim 1, wherein the rod portions (12) have polygonal shapes, and one side of a polygonal-shaped rod portion (12) included in one unit (20) and one side of a polygonal-shaped rod portion (12) included in another unit (20) are in a same virtual straight line (X1, X2).

9. A top sheet (3) that is liquid permeable and for use in an absorbent article (1), the top sheet (3) comprising,
welded portions (10) formed on at least a part of the top sheet (3),
wherein the welded portions (10) constitute a plurality of units (20), each of the units (20) including a plurality of rod portions (12) that are arranged radially in planar view,
a diameter of a smallest virtual circle (C) inscribed in the rod portions (12) included in each of the units (20) is smaller than a distance between adjacent units (20),
**characterized in that**
a short side (124A) of a first rod portion (12A) included in a first unit (20A) of the plurality of units (20) and a short side (124B) of a first rod portion (12B) of a second unit (20B) of the plurality of units (20), the second unit (20B) being not the first unit (20A), are in a first virtual straight line (X1), and
a short side (124A') of a second rod portion (12A') included in the first unit (20A) and a short side (124B') of a second rod portion (12B') are in a second virtual straight line (X2), such that
when a force causing the top sheet (3) to twist is applied, a plurality of units (20) can deform in the same direction along the first or second virtual straight line.

## Patentansprüche

1. Absorbierender Artikel (1), aufweisend:
eine Decklage (3); eine flüssigkeitsundurchlässige Rückseitenlage (2); und einen absorbierenden Körper (4), der zwischen der Decklage (3) und der Rückseitenlage (2) eingefügt ist,
wobei Schweißabschnitte (10) an mindestens einem Teil der Decklage (3) gebildet sind,
die Schweißabschnitte (10) eine Vielzahl von Einheiten (20) bilden, wobei jede der Einheiten (20) Stababschnitte (12) enthält, die in Draufsicht radial angeordnet sind,
ein Abstand zwischen benachbarten Einheiten (20) größer als ein Durchmesser eines kleinsten virtuellen Kreises (C) ist, der in die Stababschnitte (12) eingeschrieben ist, die in jeder der Einheiten (20) enthalten sind,
**dadurch gekennzeichnet, dass**
eine kurze Seite (124A) eines ersten Stababschnitts (12A), der in einer ersten Einheit (20A) der Vielzahl von Einheiten (20) enthalten ist, und eine kurze Seite (124B) eines ersten Stababschnitts (12B) einer zweiten Einheit (20B) der Vielzahl von Einheiten (20), wobei die zweite Einheit (20B) nicht die erste Einheit (20A) ist, in einer ersten virtuellen Geraden (X1) liegen, und
eine kurze Seite (124A') eines zweiten Stababschnitts (12A'), der in der ersten Einheit (20A) enthalten ist, und eine kurze Seite (124B') eines zweiten Stababschnitts (12B') in einer zweiten virtuellen Geraden (X2) liegen, so dass,
wenn eine Kraft aufgebracht wird, welche die Decklage (3) sich verziehen lässt, eine Vielzahl von Einheiten (20) sich in derselben Richtung entlang der ersten oder zweiten virtuellen Geraden verformen können.

2. Absorbierender Artikel (1) nach Anspruch 1, wobei die Decklage (3) gebildet wird, indem eine erste Decklage (3A) und eine zweite Decklage (3B) laminiert werden, wobei sich die erste Decklage (3A) auf einer Hautseite und die zweite Decklage (3B) auf einer Unterkleidungsseite befindet, wenn der absorbierende Artikel (1) getragen wird.

3. Absorbierender Artikel (1) nach Anspruch 1, wobei der Durchmesser des virtuellen Kreises 0,5 mm bis 5,0 mm beträgt.

4. Absorbierender Artikel (1) nach Anspruch 1, wobei die Anzahl der Stababschnitte (12), die in jeder der Einheiten (20) enthalten sind, 2 bis 5 beträgt.

5. Absorbierender Artikel (1) nach Anspruch 1, wobei jeder der Stababschnitte (12) ein Aspektverhältnis von 2 bis 10 hat.

6. Absorbierender Artikel (1) nach Anspruch 1, wobei die Einheiten (20) in einem versetzten Muster angeordnet sind.

7. Absorbierender Artikel (1) nach Anspruch 1, wobei jeder der Stababschnitte (12) eine Deltoidform hat, deren kurze Seiten (124) einem Mittelpunkt einer entsprechenden Einheit (20) zugewandt sind.

8. Absorbierender Artikel (1) nach Anspruch 1, wobei die Stababschnitte (12) polygonale Formen haben und eine Seite eines polygonal geformten Stababschnitts (12), der in einer Einheit (20) enthalten ist, und eine Seite eines polygonal geformten Stababschnitts (12), der in einer anderen Einheit (20) enthalten ist, in derselben virtuellen Geraden (X1, X2) liegen.

9. Decklage (3), die flüssigkeitsdurchlässig und zur Verwendung in einem absorbierenden Artikel (1) gedacht ist, wobei die Decklage (3) aufweist:
Schweißabschnitte (10), die an mindestens eine Teil der Decklage (3) gebildet sind,
wobei die Schweißabschnitte (10) eine Vielzahl von Einheiten (20) bilden, wobei jede der Einheiten (20) eine Vielzahl von Stababschnitten (12) enthält, die in Draufsicht radial angeordnet sind,
wobei ein Durchmesser eines kleinsten virtuellen Kreises (C), der in die Stababschnitte (12) eingeschrieben ist, die in jeder der Einheiten (20) enthalten sind, kleiner als ein Abstand zwischen benachbarten Einheiten (20) ist,
**dadurch gekennzeichnet, dass**
eine kurze Seite (124A) eines ersten Stababschnitts (12A), der in einer ersten Einheit (20A) der Vielzahl von Einheiten (20) enthalten ist, und eine kurze Seite (124B) eines ersten Stababschnitts (12B) einer zweiten Einheit (20B) der Vielzahl von Einheiten (20), wobei die zweite Einheit (20B) nicht die erste Einheit (20A) ist, in einer ersten virtuellen Geraden (X1) liegen, und
eine kurze Seite (124A') eines zweiten Stababschnitts (12A'), der in der ersten Einheit (20A) enthalten ist, und eine kurze Seite (124B') eines zweiten Stababschnitts (12B') in einer zweiten virtuellen Geraden (X2) liegen, so dass,
wenn eine Kraft aufgebracht wird, welche die Decklage (3) sich verziehen lässt, eine Vielzahl von Einheiten (20) sich in derselben Richtung entlang der ersten oder zweiten virtuellen Geraden verformen können.

## Revendications

1. Article absorbant (1) comprenant :
une feuille supérieure (3) ; une feuille arrière (2) imperméable aux liquides ; et un corps absorbant (4) interposé entre la feuille supérieure (3) et la feuille arrière (2),
sachant que des parties soudées (10) sont formées sur au moins une partie de la feuille supérieure (3),
les parties soudées (10) constituent une pluralité d'unités (20), chacune des unités (20) incluant des parties tige (12) qui sont agencées radialement en vue en plan,
une distance entre des unités (20) adjacentes est plus grande qu'un diamètre d'un plus petit cercle virtuel (C) inscrit dans les parties tige (12) incluses dans chacune des unités (20),
**caractérisé en ce que**
un petit côté (124A) d'une première partie tige (12A) incluse dans une première unité (20A) de la pluralité d'unités (20) et un petit côté (124B) d'une première partie tige (12B) d'une deuxième unité (20B) de la pluralité d'unités (20), la deuxième unité (20B) n'étant pas la première unité (20A), se trouvent sur une première ligne droite virtuelle (X1), et
un petit côté (124A') d'une deuxième partie tige (12A') incluse dans la première unité (20A) et un petit côté (124B') d'une deuxième partie tige (12B') se trouvent sur une deuxième ligne droite virtuelle (X2), de telle sorte que
lorsqu'une force faisant se tordre la feuille supérieure (3) est appliquée, une pluralité d'unités (20) peut se déformer dans la même direction le long de la première ou deuxième ligne droite virtuelle.

2. L'article absorbant (1) selon la revendication 1, sachant que la feuille supérieure (3) est formée par stratification d'une première feuille supérieure (3A) et d'une deuxième feuille supérieure (3B), la première feuille supérieure (3A) étant située d'un côté peau et la deuxième feuille supérieure (3B) étant située d'un côté sous-vêtement lorsque l'article absorbant (1) est porté.

3. L'article absorbant (1) selon la revendication 1, sachant que le diamètre du cercle virtuel est de 0,5 mm à 5,0 mm.

4. L'article absorbant (1) selon la revendication 1, sachant que le nombre des parties tige (12) incluses dans chacune des unités (20) est de 2 à 5.

5. L'article absorbant (1) selon la revendication 1, sachant que chacune des parties tige (12) a un rapport d'aspect de 2 à 10.

6. L'article absorbant (1) selon la revendication 1, sachant que les unités (20) sont agencées selon un motif étagé.

7. L'article absorbant (1) selon la revendication 1, sachant que chacune des parties tige (12) a une forme de cerf-volant dont les petits côtés (124) sont tournés vers un centre d'une unité (20) correspondante.

8. L'article absorbant (1) selon la revendication 1, sachant que les parties tige (12) ont des formes polygonales, et un côté d'une partie tige (12) de forme polygonale incluse dans une unité (20) et un côté d'une partie tige (12) de forme polygonale incluse dans une autre unité (20) se trouvent sur une même ligne droite virtuelle (X1, X2).

9. Feuille supérieure (3) qui est perméable aux liquides et destinée à être utilisée dans un article absorbant (1), la feuille supérieure (3) comprenant
des parties soudées (10) formées sur au moins une partie de la feuille supérieure (3),
sachant que les parties soudées (10) constituent une pluralité d'unités (20), chacune des unités (20) incluant une pluralité de parties tige (12) qui sont agencées radialement en vue en plan,
un diamètre d'un plus petit cercle virtuel (C) inscrit dans les parties tige (12) incluses dans chacune des unités (20) est plus petit qu'une distance entre des unités (20) adjacentes,
**caractérisé en ce que**
un petit côté (124A) d'une première partie tige (12A) incluse dans une première unité (20A) de la pluralité d'unités (20) et un petit côté (124B) d'une première partie tige (12B) d'une deuxième unité (20B) de la pluralité d'unités (20), la deuxième unité (20B) n'étant pas la première unité (20A), se trouvent sur une première ligne droite virtuelle (X1), et
un petit côté (124A') d'une deuxième partie tige (12A') incluse dans la première unité (20A) et un petit côté (124B') d'une deuxième partie tige (12B') se trouvent sur une deuxième ligne droite virtuelle (X2), de telle sorte que
lorsqu'une force faisant se tordre la feuille supérieure (3) est appliquée, une pluralité d'unités (20) peut se déformer dans la même direction le long de la première ou deuxième ligne droite virtuelle.
